# EUROPÄISCHE PATENTANMELDUNG

(11) **EP 3 034 129 A1**
(43) Veröffentlichungstag der Anmeldung: **22.06.2016**
(21) Anmeldenummer: 14198155.5
(22) Anmeldetag: 16.12.2014
(51) Int. Cl.: A61P 35/00, A61K 36/28

(54) **Verfahren zur Herstellung eines Echinacea purpura Trockenpresssaftes und Verwendung desselben zur Behandlung von Equinem Sarkoid**

(71) Anmelder: Agon Pharma GmbH, 73240 Wendlingen (DE)
(72) Erfinder: Stephan, Günter, 70469 Stuttgart (DE)
(74) Vertreter: Stork Bamberger

(57) **Zusammenfassung**

Ein Trockenpresssaft aus Echinacea purpurea, hergestellt aus einem Echinacea purpurea-Presssaft, indem der Echinacea purpurea-Presssaft bei maximal 70°C zu einem Droge-Extrakt-Verhältnis von mindestens 30 : 1 getrocknet wird, erweist sich als hochwirksam zur Behandlung von Equinem Sarkoid. Der Trockenpresssaft wird vorzugsweise in Alkohol gelöst durch 1-5 maliges Aufsprühen am Tag appliziert. Da der Wirkstoff des erfindungsgemäßen Presssaftes, das Echinacea purpurea, und dessen topische Anwendung nicht auf der Dopingliste vermerkt sind, kann der erfindungsgemäße Presssaft bedenkenlos zur Behandlung betroffener Sportpferde auch während der Trainings- und Wettkampfphasen eingesetzt werden.

## Beschreibung

Das Equine Sarkoid ist ein Hauttumor, der bei Pferden keine Metastasen ausbildet und deshalb auch nicht lebensbedrohend ist. Allerdings ist ein Sarkoid unansehnlich und störend, wenn es zum Beispiel in der Sattellage oder im Bereich des Sattelgurts auftritt. Es gibt unterschiedliche Formen des Sarkoids von klein und warzenartig bis hin zu großflächigen kugeligen Tumoren, die häufig am Ohrrand, an der Achsel, am Genitalbereich, am Bauch oder am Auge auftreten. Die Tumoren finden sich unabhängig von Pferderasse und Fellfarbe; insbesondere erkranken Wallache im Alter von 3 bis 8 Jahren. Bedauerlicherweise ist das Equine Sarkoid mit einer hohen Rückfallquote behaftet.

Wodurch das Equine Sarkoid hervorgerufen wird, ist noch nicht geklärt. Nach derzeitigem Kenntnisstand sollen bovine Papillomviren (BPV) am Auftreten des Equinen Sarkoids beteiligt sein. Allerdings bedeutet das Vorhandensein von Papillomviren beim Pferd nicht automatisch das Auftreten des Equinen Sarkoids. Der Immunstatus und auch genetische Prädisposition scheinen eine Rolle zu spielen.

Für die Behandlung des Equinen Sarkoids kommen bislang insbesondere die nur für den Menschen zugelassene Präparate Aciclovir-Salbe und Veregen^{®} (eingetragene Marke der Firma MediGene AG) Salbe zur Anwendung. Ersteres ist ein Arzneistoff zur Behandlung von durch Viren der Herpesfamilie hervorgerufene Krankheiten und letzteres ein Trockenextrakt aus Grüntee. Beide Präparate haben den Nachteil, dass sie bei einer Anwendung am Pferd teuer sind und dass - jedenfalls beim Menschen - ein hohes Nebenwirkungspotenzial beschreiben ist. Darüber hinaus sind die Präparate entweder apotheken- oder sogar verschreibungspflichtig.

Weitere Behandlungswege sind die chirurgische Entfernung, die sich aufgrund des invasiven Wachstums der Tumore häufig sehr schwierig gestaltet, Kryotherapie, Laser-Therapie mittels CO₂-Laser oder Neodym-YAG-Laser, Hochfrequenz-Chirurgie, Radiotherapie, Chemotherapie oder Immuntherapie mit BCG (Bacillus Calmette Guerin). Allen Behandlungsmethoden ist gemein, dass sie eine hohe Rezidivrate aufweisen. Für Sportpferde ist außerdem die Frage des Dopings relevant. Zahlreiche der genannten Präparate oder Verfahren stehen für Sportpferde auf der Liste der verbotenen Substanzen bzw. der verbotenen Methoden, so dass eine Anwendung während der Wettkampfzeit nicht möglich ist.

Es besteht daher das Bedürfnis, nach einer Methode zur Behandlung des Equinen Sarkoids, die leicht anzuwenden ist, möglichst wenig Nebenwirkungen aufweist, nicht unter Dopingsubstanzen oder im Wettkampf verbotene Methoden fällt, kostengünstig ist und mit einer geringen Rückfallrate behaftet ist.

Aus der EP 0 918 458 ist bekannt, dass eine Zusammensetzung aus einem phytochemischen Konzentrat aus Echinacea purpurea und Benzalkoniumchlorid zur Behandlung von Viren der Herpesfamilie eingesetzt werden kann. Das Präparat wurde auch für die Behandlung eines Pferdes getestet, das am Maul eine durch Papillomviren hervorgerufene Warze aufwies. Die Warze bildete sich nach topischer Behandlung über mehrere Tage zurück und fiel ab.

Wie in der EP 0 918 458 ausgeführt und durch Versuche belegt wurde, beruht die Wirksamkeit des dort offenbarten Präparates auf der Kombination von phytochemischem Echinacea purpurea-Konzentrat und dem Benzalkoniumchlorid. Vergleichstest mit den jeweiligen Einzelbestandteilen zeigten keine oder nur eine sehr schwache Wirkung, so dass der Wirkmechanismus mit einem Zusammenspiel der beiden Komponenten erklärt wird. Verwendet wird ein Echinacea-Extrakt, wobei nicht näher ausgeführt wird, wie der Extrakt gewonnen wird.

Die Wirkung von Extrakten aus Echinacea purpurea (Purpur-Sonnenhut oder Roter Scheinsonnenhut) ist seit langem bekannt. So kommen solche Extrakte zur Behandlung rezidivierender Infekte im Bereich der Atemwege und der ableitenden Harnwege zum Einsatz. Auch eine gewisse antivirale Wirkung ist bekannt. Vimalanathan et al. haben die Wirkung verschiedener Extrakte aus unterschiedlichen Bestandteilen des Echinacea purpurea (gesamte Pflanze, Blätter und Stängel, Blüte bzw. Wurzel) gegen unterschiedlichen Virenarten untersucht (Pharm. Biology, 2005, Vol. 43, No. 9, S. 740-745 und S. 791-796). Dabei wurde gefunden, dass sich die Wirksamkeit von Echinacea purpurea gegen Herpex Simplex Viren und Influenza Viren von der gegen Rhinoviren sehr stark unterscheidet. Während Echinacea purpurea gegen die beiden erstgenannten Viren eine gute Wirksamkeit zeigt, ist dies für die Rhinoviren nicht der Fall. Eine mögliche Erklärung könnte sein, dass es sich im Falle der beiden ersten Viren um behüllte Viren handelt, während Rhinoviren unbehüllte Viren sind. Auch das Papillomvirus ist ein unbehülltes Virus.

Im Rahmen der vorliegenden Erfindung wurde die Verwendung von Echinacea-Extrakten zur Behandlung von Equinem Sakoid untersucht. Dabei stellten sich bei unterschiedlichen Extrakten zunächst nicht die gewünschten Wirkungen ein. Ein Presssaft von Echinacea purpurea brachte zwar zunächst eine gewisse Besserung, erzielte jedoch keine nachhaltige Wirkung.

Überraschenderweise wurde im Rahmen der vorliegenden Erfindung gefunden, dass ein Trockenpresssaft aus einem Echinacea purpurea -Presssaft, der bei maximal 70°C zu einem Droge-Extrakt-Verhältnis von mindestens 30 : 1 sprühgetrocknet wird, hoch wirksam zur Behandlung des Equinen Sarkoids ist.

Während ein einfacher Presssaft aus Echinacea purpurea nur unbefriedigende Ergebnisse liefert, gelingt es, mit Hilfe des erfindungsgemäßen Trockenpresssaftes aus Echinacea purpurea das Equine Sarkoid nachhaltig zu behandeln. Es ist eine vollständige Heilung zu beobachten, und zwar innerhalb vergleichsweise kurzer Zeit. Die Bildung von Rezidiven ist zurückgedrängt.

Da der Wirkstoff des erfindungsgemäßen Trockenpresssaftes, das Echinacea purpurea, und dessen topische Anwendung nicht auf der Dopingliste vermerkt sind, kann der erfindungsgemäße Trockenpresssaft bedenkenlos zur Behandlung betroffener Sportpferde auch während der Trainings- und Wettkampfphasen eingesetzt werden. Auch sind für Echinacea purpurea bei äußerer Anwendung keinerlei Nebenwirkungen oder Gegenanzeigen bekannt, so dass das Präparat unbedenklich eingesetzt werden kann.

Vorzugsweise sind in dem Trockenpresssaft neben den Wirkstoffen des Echinacea purpurea keine weiteren Wirkstoffe enthalten. Die Wirkung gegen das Equine Sarkoid rührt somit allein von dem Echinace purpurea-Trockenpresssaft her, nicht von weiteren Wirkstoffen. Der Presssaft enthält darüber hinaus lediglich Hilfsstoffe wie Maltodextrin, Ethanol oder Wasser.

Zur Herstellung des Trockenpresssaftes wird ein Echinacea purpurea Presssaft bei Temperaturen von maximal 70°C getrocknet. Dazu wird der Presssaft vorzugsweise zunächst mit Ethanol versetzt, um eine Gärung zu vermeiden. Vorzugsweise erfolgt die Sprühtrocknung bei zwischen 60 und 70°C. Der so entstehende Trockenpresssaft weist ein Droge-Extrakt-Verhältnis von mindestens 30 : 1 auf. Ein Droge-Extrakt-Verhältnis von 30 : 1 bedeutet, dass aus 30 Teilen Droge ein Teil Extrakt gewonnen wird. Vorzugsweise liegt das Droge-Extrakt-Verhältnis des Trockenpresssaftes bei mindestens 40 : 1, insbesondere bei mindestens 45 : 1 und ganz besonders bevorzugt bei mindestens 50 : 1.

Die Herstellung des Echinacea purpurea-Presssaftes erfolgt vorzugsweise folgendermaßen und unter Verwendung der frisch gepflückten oberirdischen Teile von Echinacea purpurea:
Die frischen oberirdischen Teile von blühendem Echinacea purpurea werden zerkleinert und ausgepresst. Diesem nativen Saft wird Ethanol, vorzugsweise 96% Ethanol Ph.Eur., zugesetzt. Der Saft wird homogenisiert, vorzugsweise erfolgt dies durch Rühren über zwischen 10 und 60 Minuten. Der Alkoholgehalt des fertigen Presssaftes beträgt vorzugsweise zwischen 5 und 50 Vol.-%, insbesondere zwischen 10 und 40 Vol.-% und besonders bevorzugt zwischen 15 und 30 Vol.-% und ganz besonders bevorzugt zwischen 20 und 25 Vol.-%.

Statt der Verwendung aller oberirdischen Teile ist es auch möglich nur die Blütenhorizonte einzusetzen.

Das Droge-Extraktverhältnis beträgt vorzugsweise zwischen 1,5 : 1 und 3,0 : 1, insbesondere zwischen 1,7 : 1 und 2,5 : 1, besonders bevorzugt 2 : 1. Vorzugsweise wird dieser Saft gelagert, damit sich feste Substanzen absetzen können. Die Lagerzeit beträgt vorzugsweise mindestens vier Wochen, insbesondere mindestens drei Monate und besonders bevorzugt nicht mehr als 18 Monate. Nach erfolgter Sedimentation wird der Saft abfiltriert.

Für den erfindungsgemäßen Echinacea purpurea-Trockenpresssaft kann sowohl kaltgepresster wie auch heißgepresster Echinacea purpurea-Presssaft verwendet werden.

Zur Herstellung des Trockenpresssaftes wird der Presssaft vorzugsweise zunächst eingedampft und dabei aufkonzentriert. Dies kann vorzugsweise in einem Dünnschichtverdampfer erfolgen. Die Temperatur bei diesem Verdampfungsschritt beträgt maximal 70°C, insbesondere maximal 60°C und besonders bevorzugt maximal 55°C.

Für die folgende Trocknung des konzentrierten Presssaftes kann diesem in einer vorteilhaften Ausführungsform ein Hilfsstoff wie zum Beispiel Maltodextrin zugesetzt werden. Konzentrierter Presssaft und Hilfsstoff werden dann unter Rühren vermischt.

Die Trocknung des ggf. mit einem Hilfsstoff vermischten Presssaftes erfolgt bei Temperaturen von maximal 70°C, vorzugsweise maximal 65°C und insbesondere maximal 60°C. Vorteilhaft ist es ferner, wenn die Temperatur mindestens 40°C, insbesondere mindestens 50 und besonders bevorzugt mindestens 55°C beträgt.

Die Trocknung erfolgt vorzugsweise in einem Sprühtrockner als Sprühtrocknung. Auch andere Trocknungsverfahren wie Sprühbandtrocknung oder Vakuumbandtrocknung sind möglich.

Der erhaltene Trockenpresssaft wird vorzugsweise noch gemahlen und gesiebt. Man erhält ein braunes Pulver.

Der erfindungsgemäße Echinace purpurea-Trockenpresssaft weist ein sehr viel höheres Droge-Extrakt-Verhältnis auf als der Presssaft. Dieses beträgt mindestens 30 : 1, insbesondere mindestens 40 : 1, vorzugsweise mindestens 45 : 1 und besonders bevorzugt mindestens 50 : 1.

Vorzugsweise wird der Trockenpresssaft topisch appliziert. Dies erfolgt besonders geeigneter Weise, indem der Trockenpresssaft in einer Flüssigkeit, vorzugsweise Ethanol, gelöst wird. Der Alkoholgehalt der fertigen Trockenpresssaft-Lösung beträgt vorzugsweise von 5 bis 50 Vol.-%, insbesondere von 5 bis 40 Vol.-% und besonders bevorzugt von 7 bis 30 Vol.-% und ganz besonders bevorzugt 10 Vol.-%.

Der fertige Saft wird vorzugsweise durch Aufsprühen auf die betroffenen Körperteile appliziert. Die Anwendung ist somit sehr einfach.

Die Anwendung erfolgt vorzugsweise 1 bis 5 Mal pro Tag, insbesondere 2 bis 3 Mal pro Tag, durch Aufsprühen des unverdünnten Presssaftes auf die betroffenen Körperteile.

Vorzugsweise beträgt die Anwendungsdauer mindestens zehn Tage, insbesondere mindestens zwei Wochen.

### Beispiel

### Herstellung Presssaft

Echinacea purpurea-Blüten und -Kraut (oberirdische Teile der blühenden Pflanze Echinacea purpurea (L.) Moench) wurden durch Mähen und Häckseln geerntet. Nach erfolgter Überprüfung der Schnittgröße wurden 1.800 kg der frisch gepflückten oberirdischen Teile der blühenden Pflanze in einer Lochplattenmühle (Spalt 8 bis 10 mm) gemahlen und im Anschluss in einer Siebbandpresse ausgepresst. Der Flächendruck betrug im Schnitt 1.500 - 2.500 kPa, die Presszeit ca. 5 Minuten. Man erhielt 750 kg nativen Saft. Dem so erhaltenen nativen Saft wurden 170 kg Ethanol 96% Ph. Eur. zugesetzt. Der Saft wurde mit einem Rührwerk homogenisiert. Die Zeit betrug mindestens 20 Min. Der mit Ethanol stabilisierte Presssaft wurde 8 Wochen lang in Edelstahltanks gelagert. Es trat eine Sedimentation der festen Bestandteile ein. Nach erfolgter Sedimentation wurde der Saft in einem Schichtenfilter abfiltriert. Der Saft wurde in Sprühflaschen abgefüllt. Man erhielt 920 kg fertigen Saft. Das Droge-Extrakt-Verhältnis betrug 2,1 : 1.

### Herstellung Trockenpresssaft

70 kg des so erhaltenen Presssaftes wurde in einem Dünnschichtverdampfer bei 55°C und 150 mbar aufkonzentriert. Der aufkonzentrierte Presssaft wurde in einen Sprühtrockner gegeben und bei 45-60°C sprühgetrocknet. Man erhielt ein braunen Pulver. Das so erhaltene Pulver wurde gemahlen, vermischt und gesiebt (Siebgröße: 1 mm). Das Droge-Extrakt-Verhältnis des so erhaltenen Trockenpresssaftes betrug 46 : 1.

### Herstellung Lösung

300 g des gewonnenen Trockenpresssaftes wurden in einer Mischung aus 486 g (600 ml) 96 %igem Ethanol und 2,4 kg Wasser gelöst und 10 Min. gemischt. Man erhielt 3,186 kg einer 10%igen Trockenpresssaftlösung.

### Vergleichsbeispiel

Der ungetrocknete Presssaft wurde einer fünfjährigen Stute mit einem Equinen Sarkoid von ca. 5,5 cm Durchmesser am Schenkel durch 2-3-maliges Aufsprühen pro Tag über 25 Tage appliziert. Es konnte zunächst eine Besserung beobachtet werden. Allerdings traten nach Beendigung der Behandlung Rezidive, also erneut Sarkome, auf.

### Beispiel

Die Trockenpresssaftlösung wurde einer sechsjährigen Rappstute mit einem Equinen Sarkoid von ca. 6 cm Durchmesser am Schenkel durch 2-3-maliges Aufsprühen pro Tag über 25 Tage appliziert.

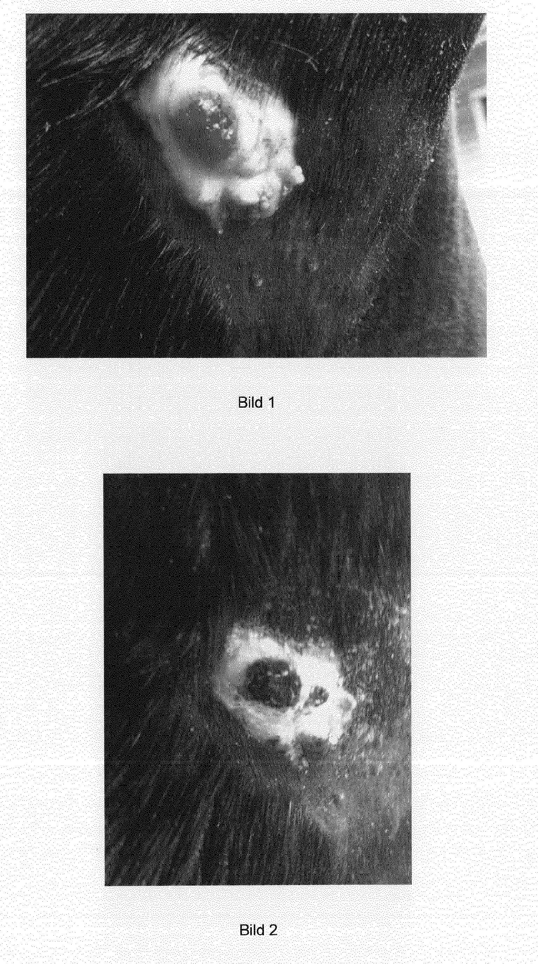

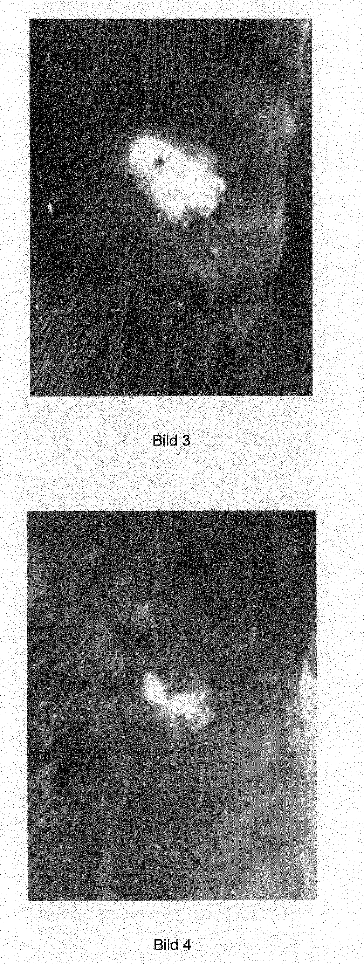

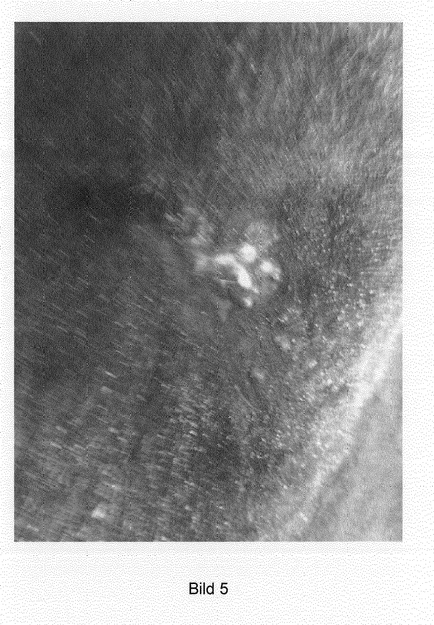

Schon kurz nach der ersten Anwendung konnte eine Sekretabsonderung und dann eine deutliche Verkleinerung des Sarkoms bis zur Abheilung beobachtet werden. Die beigefügten Abbildungen 1-5 dokumentieren den Heilungsverlauf, wobei das erste Bild das unbehandelte Sarkom und Bild 4 die fast vollständig abgeheilte Wunde nach 25 Tagen zeigt. Bild 5 ist eine Aufnahme, die nach weiteren zwei Wochen gemacht wurde und zeigt, dass auch bereits wieder Fell über die geheilte Wunde zu wachsen begonnen hat. Auch nach einem Zeitraum von vier Monaten konnten keine Rezidive beobachtet werden.

## Patentansprüche

1. Verfahren zur Herstellung eines Echinacea purpurea-Trockenpresssaftes aus einem Echinacea purpurea-Presssaft, **dadurch gekennzeichnet, dass** der Echinacea purpurea-Presssaft bei maximal 70°C zu einem Droge-Extrakt-Verhältnis von mindestens 30 : 1 getrocknet wird.

2. Verfahren nach Anspruch 1, **dadurch gekennzeichnet, dass** das Droge-Extrakt-Verhältnis mindestens 40 : 1, insbesondere mindestens 50 : 1, beträgt.

3. Verfahren nach Anspruch 1 oder 2, **dadurch gekennzeichnet, dass** der Echinacea purpurea-Presssaft bei mindestens 40°C, insbesondere mindestens 45°C, getrocknet wird.

4. Verfahren nach einem der Ansprüche 1 bis 3, **dadurch gekennzeichnet, dass** der Echinacea purpurea-Presssaft bei mindestens 50°C, insbesondere mindestens 55°C, und höchstens 60°C getrocknet wird.

5. Verfahren nach einem der Ansprüche 1 bis 4, **dadurch gekennzeichnet, dass** die Trocknung als Sprühtrocknung erfolgt.

6. Verfahren nach einem der Ansprüche 1 bis 5, **dadurch gekennzeichnet, dass** dem Presssaft vor der Sprühtrocknung ein Hilfsstoff zugesetzt wird.

7. Verfahren nach einem der Ansprüche 1 bis 6, **dadurch gekennzeichnet, dass** der Echinacea purpurea-Presssaft aus den oberirdischen Teilen der blühenden Pflanze gewonnen wird.

8. Echinacea purpurea-Trockenpresssaft erhältlich nach dem Verfahren nach einem der Ansprüche 1 bis 7.

9. Verwendung des Echinacea purpurea-Trockenpresssaftes nach Anspruch 8 zur Herstellung eines Arzneimittels oder einer pharmazeutischen Zusammensetzung zur Behandlung von Equinem Sarkoid.

10. Verwendung nach Anspruch 9, **dadurch gekennzeichnet, dass** man den Echinacea purpurea-Trockenpresssaft zu einer für die topische Anwendung geeigneten Dosierungsform konfektioniert.

11. Verwendung nach Anspruch 9 oder 10, **dadurch gekennzeichnet, dass** der Echinacea purpurea-Trockenpresssaft als 10%ige ethanolische Lösung konfektioniert wird.

12. Verwendung nach einem der Ansprüche 9 bis 11, **dadurch gekennzeichnet, dass** der Echinacea purpurea-Trockenpresssaft als Spray konfektioniert wird.

13. Verwendung nach Anspruch 12, **dadurch gekennzeichnet, dass** das Spray 1 bis 5 Mal, insbesondere 2 bis 3 Mal, am Tag auf die betroffenen Körperteile appliziert wird.
